**Europäisches Patentamt**

**European Patent Office**

**Office européen des brevets**

(11) Veröffentlichungsnummer: **0 260 611 B1**

## EUROPÄISCHE PATENTSCHRIFT

(45) Veröffentlichungstag der Patentschrift: **08.01.92**

(51) Int. Cl.5: **C12N 9/02**, //C12P7/40, C12P41/00

(21) Anmeldenummer: 87113280.9

(22) Anmeldetag: **11.09.87**

(54) **Verfahren zur Durchführung enzymatischer Oxidationen.**

(30) Priorität: **13.09.86 DE 3631228**

(43) Veröffentlichungstag der Anmeldung:
**23.03.88 Patentblatt 88/12**

(45) Bekanntmachung des Hinweises auf die
Patenterteilung:
**08.01.92 Patentblatt 92/02**

(84) Benannte Vertragsstaaten:
**AT BE CH DE ES FR GB IT LI NL SE**

(56) Entgegenhaltungen:

**ANGEWANDTE CHEMIE**

**APPLIED MICROBIOL. BIOTECHNOL**

**ANGEWANDTE CHEMIE**

**BIOCHEMICAL JOURNAL**

**CHEMICAL SOCIETY REVIEWS**

**PATENT ABSTRACTS OF JAPAN**

(73) Patentinhaber: **BASF Aktiengesellschaft
Carl-Bosch-Strasse 38
W-6700 Ludwigshafen(DE)**

(72) Erfinder: **Simon, Helmut, Prof.Dr.
Egilbertstrasse 31
W-8050 Freising(DE)**
Erfinder: **Guenther, Helmut, Dr.
Amperblick 11
W-8051 Haag(DE)**

Angewandte Chemie, Band 99, Nr 2, Februar 1987, Seiten 139-141 Weinheim, DE, H. Skopan et al "Ein Biokatalysator zur Herstellung von (R)- und (S)-2-Hydroxycarbonsäuren" insgesamt

Applied Microbiol. Biotechnol, Band 26, Nr 3, Juni 1987, Seiten 263-267, Berlin, DE. S. nagata et al "On a viologen-dependent pyridine nucleotide oxidoreductase from a thermophilic Bacillus " Zusammenfassung

Angewandte Chemie, Band 97, Juli 1985, Seiten 541-555, Weinheim, DE, H. Simon et al "Chirale Verbindungen durch biokatalytische Reduktionen" Zusammenfassung

Biochemical Journal, Band 205, Nr 1, 1982, Seiten 235-238, London, GB, I.V. Fry et al "Kinetics of leaf nitrite reductase with Methyl Viologen and ferredoxin under controlled redox conditions"

Chemical Society Reviews, Band 10, 1981, Seiten 49-82, London, GB, C.L. Bird et al "Electrochemistry of the viologens"

Patent abstract of Japan, Band 5,Nr 20 (C42)(692), Februar 1981 & JP A 55 147260 (Rikagaku Kenkyusho), 17.11.80

**Beschreibung**

Die Erfindung betrifft ein Verfahren zur Durchführung enzymatischer Oxidationen.

Es ist bekannt, 2-Oxocarbonsäuren mit Hilfe von Mikroorganismen zu reduzieren, denen die Reduktionsäquivalente über einen Elektronenüberträger, der elektrochemisch regeneriert wird, zur Verfügung gestellt werden (EP-OS 99.157, Angew. Chem. 97, 541 (1985)). Als Elektronenüberträger werden insbesondere Methyl- oder Benzylviologen verwendet. Bisher war es nicht möglich, die Reduktion von α-Oxosäurenumzukehren, selbst wenn man das eventuell in geringer Menge entstehende reduzierte Viologen mit einem stark elektropositiven Oxidationsmittel - wie rotes Blutlaugensalz - quantitativ reoxidierte (Dissertation S. Neumann, Technische Universität München, 1985).

Es wurde nun gefunden, daß es mit bestimmten Viologenen möglich ist Oxidationsreaktionen durchzuführen.

Gegenstand der Erfindung ist ein Verfahren zur Durchführung enzymatischer Oxidationen durch Übertragung von Elektronen von dem zu oxidierenden Substrat auf einen Elektronenakzeptor in Gegenwart eines Redoxenzyms, dadurch gekennzeichnet, daß man die Elektronen vom Redoxenzym mit Hilfe von CAV oder CYV auf den Elektronenakzeptor überträgt.

CAV ist das Viologen 1,1′-Dicarbamoylmethyl-4,4′-dipyridinium-Dikation und CYV das Viologen 1,1′-Dicyanomethyl-4,4′-dipyridinium-Dikation (vgl. Chem. Soc. reviews 10 (1981) 49-82).

Reduziertes CAV und CYV können elektrochemisch an Anoden elektrochemischer Zellen reoxidiert werden können. Besonders geeignet für diese Oxidation sind Anoden aus Silber, Quecksilber oder verschiedenen Formen von Kohlenstoff.

Die Reoxidation des reduzierten CAV bzw. CYV ist auch mit Luftsauerstoff möglich. Das ist besonders überraschend in Gegenwart von ganzen Zellen oder Rohextrakten von Proteus vulgaris, da die in ihren enthaltene Oxidoreduktase sehr Sauerstoff-empfindlich ist, wenn sie als Reduktase verwendet wird (Dissertation S. Neumann, Technische Universität München). Das reduzierte CAV bzw. CYV kann auch mit Elektronenakzeptoren, wie rotem Blutlaugensalz oder anderen Eisen(III)-Verbindungen sowie Wasserstoffperoxid, reoxidiert werden.

Das neue Verfahren eignet sich besonders gut zur Oxidation von α-Hydroxycarbonsäuren zu den entsprechenden Ketocarbonsäuren. Die 2-Oxosäurereduktase dehydriert in Gegenwart von CAV oder CYV nur die R-Form einer α-Hydroxycarbonsäure. So kann man den R-Antipoden in dem Gemisch oxidieren und das Oxidationsprodukt⁻ (z.B. die entsprechende Ketosäure) aus dem Gemisch entfernen oder gegebenenfalls anschließend durch Änderung der angelegten Spannung und Austausch der Enzyme in die gewünschte Verbindung überführen. Weiter lassen sich auch komplexe Naturstoffe, wie meso-Weinsäure oder Gluconsäure, selektiv dehydrieren.

Mit Hilfe des neuen Verfahrens kann man auch NADH bzw. NADPH zu NAD + bzw. NADP + oxidieren und letztere damit für Oxidationsreaktionen regenerieren. So läßt sich die Oxidation von NADH mit kommerziell erhältlicher Lipoamiddehydrogenase aus Schweineleber sowie einer Methylviologen-abhängigen Pyridinnukleotidreduktase aus thermophilem Bacillus DSM 406 durchführen. Mit der zuletzt genannten Pyridinnukleotidreduktase kann auch NADPH oxidiert werden. Bislang war es mit diesen Enzymen nur möglich, NADH bzw. NADPH zu regenerieren (J. Org. Chem. 46, 4100 (1981), Angew. Chem. 97, 541 (1985)).

Der Vorteil von CAV bzw. CYV liegt besonders darin, daß sie mit Oxosäurereduktasen reagieren und daß das Gleichgewicht für die Dehydrierungsreaktion günstig liegt. Es lassen sich daher biochemische Oxidationsreaktionen mit ihrer Hilfe leicht und schnell durchführen.

Beispiel 1

30 ml Anolyt (0,1 M Kaliumphosphat pH 8,5) einer elektrochemischen Zelle mit einer Graphitelektrode, z.B. Glassy Carbon (Sigraflex® oder Sigratex®) als Anode enthalten je 150 μMol R und S-Lactat, 2 mM CAV⁺⁺ und 10 mg (Trockengewicht) Proteus vulgaris. Bei einem Potential von -440 mV gegen eine Standardcalomel-Elektrode wurden bei pH 8,5 nach der Stromkurve in 7 h 47 % des Gesamtlactats oxidiert. Laut enzymatischem Test enthielt der Ansatz 130 μMol Pyruvat und 168 μMol S-Lactat.

Nach Hitzeinaktivierung der Enzyme und Zusatz der viologenabhängigen NAD-Reduktase aus dem thermophilen Bacillus DSM 406, 1,5 mM NAD und S-Lactat-Dehydrogenase wurde die jetzt bei -640 mV betriebene Zelle benutzt, um das Pyruvat zu reduzieren. Man erhielt insgesamt 252 μMol S-Lactat.

Dasselbe Ergebnis erhält man, wenn die Reaktion mit CYV bei pH 7,0 durchführt.

Beispiel 2

In 55 ml Anolyt (0,1 M Kaliumphosphat pH 8,5) einer elektrochemischen Zelle wurden 3,3 mMol (R,S)-2-Hydroxy-4-methylpentanoat bei pH 8,5 mit 1,5 mM CAV$^{++}$ und 32 mg Proteus vulgaris umgesetzt. Nach 5 h floß kein Strom mehr durch die Zelle. Die HPLC-Analyse ergab 1,62 mMol 2-Oxo-4-methylpentanoat und 1,57 mMol 2-Hydroxysäure. Nach Analyse auf einer Chiral-1-Säule von M & N, die R- und S-Hydroxycarbonsäure gut trennt, sind <2 % R-Enantiomeres vorhanden.

Beispiel 3

In Warburggefäßen wurden bei 35°C unter Luft folgende Ansätze geschüttelt: 3,0 ml Tris•HCl Puffer pH 8,5, 20 mg P. vulgaris (Trockengewicht), 2 mM CAV$^{++}$, 100 μMol von racemischen Mischungen von Hydroxysäuren. Nach 4 h wurden die Reaktionen beendet. Die folgende Tabelle zeigt die erhaltenen Ergebnisse.

Tabelle

| Ausgangsmaterial | Endprodukt |
|---|---|
| (R,S)-Lactat | 58,0 μMol S-Lactat[x) |
| (R,S)-Phenyllactat | 53,3 μMol S-Phenyllactat |
| | 46,3 μMol Phenylpyruvat |
| (R,S)-Hydroxybutyrat | 50,2 μMol S-Hydroxybutyrat |
| | 49,3 μMol Ketobutyrat |
| (R,S)-2-Hydroxyglutarat | 58,0 μMol S-Hydroxyglutarat[x) |
| (R,S)-2-Hydroxy-3-methylbutanoat | 49,7 μMol S-Hydroxyverbindung |
| | 48,0 μMol 2-Keto-3-methylbutyrat |

[x) Die entstehende Ketosäure wird von den Zellen metabolisiert.

Beispiel 4

In 2,5 ml 0,08 M Kaliumphosphatpuffer pH 8,35 wurden inkubiert: 40 mM (S)-Lactat, 2 mM CAV$^{++}$, 0,95 mM NADH, 95 U (S)-Lactatdehydrogenase aus Säugetier und 2,4 U Viologen-abhängige NAD(P)H Oxidoreduktase aus Bacillus DSM 406. Der Ansatz wurde in einer Sauerstoffatmosphäre bei 35°C geschüttelt. Nach 22 h ließen sich ca. 90 % des erwarteten Pyruvats enzymatisch bestimmen. Die Aktivität der Lactatdehydrogenase betrug 40 % der ursprünglichen.

Ein entsprechender Versuch mit (R)-Lactat und (R)-Lactatdehydrogenase aus Lactobacillus leichmannii brachte das gleiche Ergebnis. Die restliche Aktivität der Lactatdehydrogenase betrug 60 %. Wurden die Versuche in Gegenwart von ca. 13.000 U Katalase durchgeführt, ergaben sich keine signifikant verschiedenen Ergebnisse.

**Patentansprüche**

1.	Verfahren zur Durchführung enzymatischer Oxidationen durch Übertragung von Elektronen von dem zu oxidierenden Substrat auf einen Elektronenakzeptor in Gegenwart eines Redoxenzyms, dadurch gekennzeichnet, daß man die Elektronen vom Redoxenzym mit Hilfe der in katalytischen Mengen eingesetzten Mediatoren 1,1'-Dicarbamoylmethyl-4,4'-dipyridinium-Dikation oder 1,1'-Dicyanomethyl-4,4'-dipyridinium-Dikation auf einen Elektronenakzeptor überträgt.

**Claims**

1.	A process for carrying out enzymatic oxidations by transferring electrons from the substrate to be oxidized to an electron acceptor in the presence of a redox enzyme, wherein the electrons are transferred from the redox enzyme to an electron acceptor with the aid of the mediator 1,1'-dicarbamoylmethyl-4,4'-dipyridinium dication or 1,1'-dicyanomethyl-4,4'-dipyridinium dication used in catalytic amounts.

**Revendications**

1. Procédé pour effectuer des oxydations enzymatiques en transférant des électrons du substrat à oxyder à un accepteur d'électrons, en présence d'une enzyme d'oxydoréduction, caractérisé par le fait que l'on transfère les électrons à l'aide des médiateurs dication 1,1'-dicarbamoylméthyl-4,4'-dipyridinium ou dication 1,1'-dicyanométhyl-4,4'-dipyridinium, utilisés en quantités catalytiques.